(19) Europäisches Patentamt
European Patent Office
Office européen des brevets

(11) Veröffentlichungsnummer: **0 234 329**
**A1**

# (12) EUROPÄISCHE PATENTANMELDUNG

(21) Anmeldenummer: 87101292.8

(22) Anmeldetag: 30.01.87

(51) Int.Cl.³: C 07 D 405/04
C 07 D 405/14, C 07 D 401/1- 4
C 07 D 409/14, C 07 D 471/0- 4
C 07 D 491/04, C 07 D 495/0- 4
A 61 K 31/47
//(C07D471/04, 221:00, 209:00),
(C07D491/04, 307:00, 221:00),
(C07D491/04, 317:00, 221:00),
(C07D491/04, 319:00, 221:00),
(C07D495/04, 333:00, 221:00)

(30) Priorität: 03.02.86 DE 3603194

(43) Veröffentlichungstag der Anmeldung:
02.09.87 Patentblatt 87/36

(84) Benannte Vertragsstaaten:
AT BE CH DE ES FR GB GR IT LI LU NL SE

(71) Anmelder: BOEHRINGER INGELHEIM KG
D-6507 Ingelheim am Rhein(DE)

(84) Benannte Vertragsstaaten:
BE CH DE ES FR GR IT LI LU NL SE AT

(71) Anmelder: Boehringer Ingelheim International G.m.b.H
D-6507 Ingelheim am Rhein(DE)

(84) Benannte Vertragsstaaten:
GB

(72) Erfinder: Lösel, Walter, Dr. Dipl.-Chem.
Im Herzenacker 26
D-6535 Gau-Algesheim(DE)

(72) Erfinder: Roos, Otto, Dr. Dipl.-Chem.
Elsheimer Strasse 36
D-6501 Schwabenheim(DE)

(72) Erfinder: Schnorrenberg, Gerd, Dr. Dipl.-Chem.
Ernst-Ludwig-Strasse 66a
D-6535 Gau-Algesheim(DE)

(72) Erfinder: Arndts, Dietrich, Dr.
Mühlstrasse 7
D-6531 Appenheim(DE)

(72) Erfinder: Kuhn, Franz-Josef, Dr.
Beethovenstrasse 11
D-6535 Gau-Algesheim(DE)

(72) Erfinder: Lehr, Erich, Dr.
In der Toffel 5
D-6531 Waldalgesheim(DE)

(72) Erfinder: Reichl, Richard, Dr.
Im Hippel 55
D-6535 Gau-Algesheim(DE)

(72) Erfinder: Schingnitz, Günter, Dr.
Unter den Gärten 18
D-6550 Bad Kreuznach 14(DE)

(72) Erfinder: Speck, Georg, Dr. Dipl.-Chem.
Rheinstrasse 13
D-6507 Ingelheim/Rhein(DE)

(54) **Neue 2-Amino-3-formyl-furane, Verfahren zu ihrer Herstellung und diese enthaltende pharmazeutische Zusammensetzungen.**

(57) Verbindung der Formel I

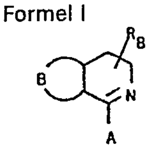

oder ihr Säureadditionssalz,
worin
A die Gruppe Ia, Ib oder Ic ist,

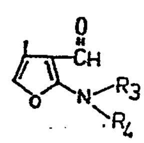

Ia :

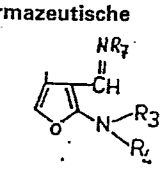

Ib

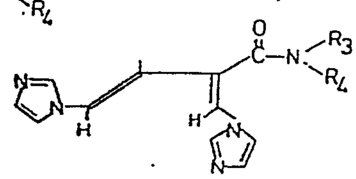

Ic

./...

EP 0 234 329 A1

B zusammen mit den beiden vicinalen C-Atomen ein aromatisches System

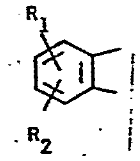 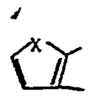 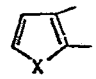 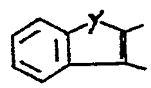 oder 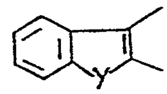

bedeutet.

Die Verbindung kann bei der Behandlung der koronaren Herzkrankheit oder des hirnorganischen Psychosyndroms Verwendung finden.

Die Erfindung betrifft Verbindungen der allgemeinen Formel I

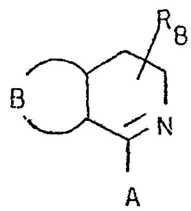

und ihre Säureadditionssalze, sowie Verfahren zu ihrer Herstellung, und diese Verbindungen enthaltende pharmazeutische Zusammensetzungen, wobei in Formel I

A die Gruppe Ia, Ib oder Ic ist,

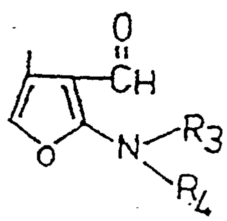

Ia

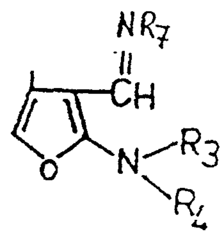

Ib

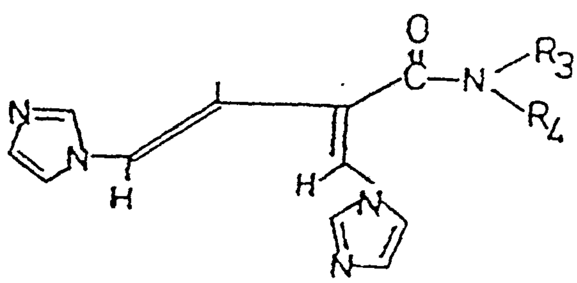

Ic

B zusammen mit den beiden vicinalen C-Atomen ein aromatisches System

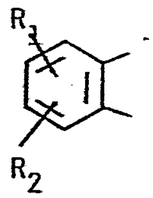 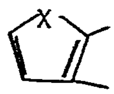 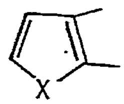 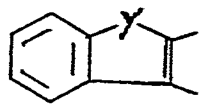 oder 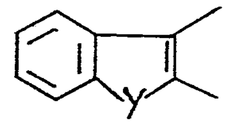

bedeutet,
worin

X S oder $NR_5$ (worin $R_5$ H, Methyl oder Benzyl ist), bedeutet;

Y S, O oder $NR_6$ (worin $R_6$ wie $R_5$ definiert ist), bedeutet;

$R_1$ und $R_2$ unabhängig voneinander Wasserstoff, $(C_1-C_4)$-alkoxy, Mercapto, Methylmercapto oder Amino oder zusammen die Gruppe 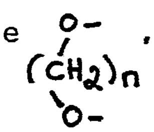 , worin n 1 oder 2 ist, bedeuten;

$R_3$ und $R_4$ unabhängig voneinander (i) Wasserstoff, oder (ii) einen gesättigten $(C_1-C_{12})$ oder ungesättigten $(C_2-C_{12})$, geradkettigen oder verzweigten oder cyclischen $(C_3-C_6)$ Kohlenwasserstoffrest, der gegebenenfalls ein- oder mehrfach durch Hydroxy, Methoxy, Amino,

($C_1$-$C_5$)-Alkylamino, Di- ($C_1$-$C_5$)-alkylamino, Phenyl (gegebenenfalls ein- oder mehrfach durch Hydroxy, Methoxy oder Halogen substituiert), oder durch einen 5- oder 6-gliedrigen gesättigten oder ungesättigten Heterocyclus (der ein oder zwei N, S oder O Heteroatome enthält, und der durch ($C_1$-$C_3$) Alkyl substituiert sein kann) substituiert ist,

oder $R_3$ und $R_4$ zusammen mit dem Stickstoffatom einen 5- oder 6-gliedrigen Ring bilden, der gegebenenfalls ein weiteres N, O oder S Heteroatom enthält, bedeuten;

$R_7$ wie $R_3$ definiert ist, und $R_8$ H, ($C_1$-$C_3$)-Alkyl oder ($C_3$-$C_6$)-Cycloalkyl oder $-SCH_3$ bedeutet.

Als Halogenatom kommen die vier üblichen Halogenatome in Frage, wobei Chlor,- Brom-, und Fluoratome bevorzugt sind.

Bevorzugt stehen $R_1$ und $R_2$ für Methoxy.

Vorzugsweise steht $R_3$ für Wasserstoff und $R_4$ für Methyl-, Ethyl-, Allyl-, Propargyl-, geradkettige oder verzweigte ($C_3$-$C_5$)-Alkyl-, Methoxy ($C_1$-$C_4$)alkyl, Hydroxy ($C_1$-$C_4$)alkyl,
oder
$R_3$ und $R_4$ für ($C_1$-$C_5$)Alkyl,
oder $R_3$ und $R_4$ zusammen mit dem Stickstoffatom für eine Pyrrolidinylgruppe.

Gruppen bevorzugter Verbindungen können wie folgt zusammengefaßt werden:

Verbindungen (I), worin B zusammen mit den vicinalen C-Atomen die Gruppe

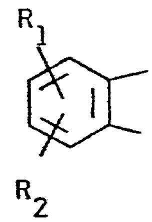

bedeutet, insbesondere Verbindungen, worin $R_1$ und $R_2$ in Position 6 beziehungsweise 7 sind, vorzugsweise Verbindungen, worin $R_1$ und $R_2$ Methoxy sind.

Verbindungen (I), worin $R_3$ für Wasserstoff und $R_4$ für Methyl-, Ethyl-, Allyl-, Propargyl-, geradkettige oder verzweigte ($C_3$-$C_5$)alkyl-, Methoxy ($C_1$-$C_4$)alkyl- oder Hydroxy ($C_1$-$C_4$)alkyl, steht,

oder

worin $R_3$ für Wasserstoff und $R_4$ für ($C_3$ bis $C_6$) Cycloalkyl oder Phenyl steht,

oder

worin $R_3$ und $R_4$ unabhängig voneinander für eine ($C_1$-$C_5$)-Alkylgruppe stehen,
insbesondere Verbindungen, worin $R_3$ für H und $R_4$ für $CH_3$ oder $(CH_3)_2$-CH-$(CH_2)_2$ - oder
$C_2H_5$-CH($CH_3$)-$CH_2$- oder $(CH_3)_3$-C- steht,

oder

$R_3$ und $R_4$ für $C_2H_5$ stehen oder zusammen die Gruppe -$(CH_2)_4$- bilden.

Verbindungen (I) worin A die Gruppe der Formel (Ia) ist.

-) Verbindungen (I), worin $R_8$ H ist.
-) Verbindungen (I), worin A die Gruppe der Formel Ib ist, worin $R_7$ einen gesättigten ($C_1$-$C_{12}$) oder ungesättigten ($C_2$-$C_{12}$), geradkettigen oder verzweigten oder cyclischen ($C_3$-$C_6$) Kohlenwasserstoffrest, der ein- oder mehrfach durch Hydroxy, Methoxy, Amino, ($C_1$-$C_5$)-Alkylamino, Di- ($C_1$-$C_5$)-alkylamino, Phenyl (gegebenenfalls ein- oder mehrfach durch Hydroxy, Methoxy oder Halogen substituiert), oder durch einen 5- oder 6-gliedrigen gesättigten oder ungesättigten Heterocyclus (der ein oder zwei N, S oder O Heteroatome enthält, und der durch ($C_1$-$C_3$)Alkyl substituiert sein kann) substituiert ist, insbesondere Verbindungen worin $R_7$ identisch mit $R_3$ oder $R_4$ derselben Verbindung ist.

Die erfindungsgemäßen Verbindungen der Formel I sind Basen und können auf übliche Weise mit anorganischen oder organischen Säuren in Säureadditionssalze überführt werden, wobei Säureadditionssalze auch durch Umsalzen aus anderen Säureadditionssalzen hergestellt werden können.

Zur Salzbildung geeignete Säuren sind beispielsweise Salzsäure, Bromwasserstoffsäure, Jodwasserstoffsäure, Fluorwasserstoffsäure, Schwefelsäure, Phosphorsäure, Salpetersäure, Essigsäure, Propionsäure, Buttersäure, Capronsäure, Valeriansäure, Oxalsäure, Malonsäure, Bernsteinsäure, Maleinsäure, Fumarsäure, Milchsäure, Weinsäure, Zitronensäure, Äpfelsäure, Benzoesäure, p-Hydroxybenzoesäure, p-Aminobenzoesäure, Phthalsäure, Zimtsäure, Salicylsäure, Ascorbinsäure, Methansulfonsäure und dergleichen.

Die Verbindungen der allgemeinen Formel I und ihre Säureadditionssalze besitzen im Tierversuch wertvolle therapeutische Eigenschaften. Sie besitzen eine deutliche cardioprotektive Wirkung, die wie folgt bestimmt wurde:

Bekanntlich ist der myocardiale $Ca^{+2}$-Gehalt ein Maß für die hypoxische bzw. durch toxische Catecholamindosen

hervorgerufene Herzschädigung (HIGGINS et al. Mol. Cell, Cardiol., 10: 427-438, 1984; NAKANISHI et al., Am. J. Physiol. 242 : 437 - 449, 1982; FLECKENSTEIN, Vorträge der Erlanger Physiol. Tagung 19700, Ed. Keidel, Springer Verlag Berlin, Heidelberg, New York, 1971). Umgekehrt ist die Inhibition der hypoxischen oder Isoprenalin-bedingten myocardialen Calziumaufnahme ein Maß für die cardioprotektive Effektivität von Calziumantagonisten (FLECKENSTEIN s.o.), von Calmodulin-Inhibitoren (HIGGINS) und anderen Pharmaka z.B. Betaadrenolytika (ARNDTS, Arzneimittelforschung 25 : 1279 - 1284, 1975).

Die cardioprotektive Wirkung wurde an wachen Ratten nach subkutaner oder peroraler Wirkstoffgabe anhand der von ARNDTS (s.o.) beschriebenen Methode festgestellt und die Wirkungsstärke der Testsubstanzen als $H_{50}$-Wert angegeben. Dieser Wert entspricht der Dosis, die die durch Gabe von 30 mg/kg s.c. Isoprenalin-bedingte myocardiale Radio-Calcium-Aufnahme zu 50 % hemmt.

Hier erwiesen sich die untersuchten neuen Verbindungen als bis zu 5 mal wirksamer als das bekannte Handelsprodukt Propranolol. In vitro-Untersuchungen an der glatten

Muskulatur (Aortenstreifen) nach der Methode von C. Van Breemen, P. Aaranson, R. Loutzenheiser und K. Meisheri (Chest 78: 1575-1655 (1980)) und von R. Casteels u. C. Droogman (J. Physiol. 317: 263 - 279 (1981)) haben ergeben, daß es sich bei den erfindungsgemäßen Verbindungen um Calciumantagonisten mit einem neuen Wirkungsmechanismus handelt.

Aufgrund dieser Befunde sollen die Verbindungen der allgemeinen Formel I beziehungsweise ihre Säureadditionssalze als Wirkstoffe für Arzneimittel gegen die koronare Herzkrankheit z.B. Angina pectoris, Herzinfarkt und Herzflimmern Verwendung finden. Außerdem verbessern sie die Gewebsdurchblutung und die Gewebssauerstoffversorgung besonders im zentralen Nervensystem. In Versuchen mit Einschränkung des Kurzzeitgedächtnisses bzw. Behinderung des Übergangs von Inhalten des Kurzzeit- in das Langzeitgedächtnis durch Gabe des muskarin-cholinergen Antagonisten Scopolamin (0,6 mg/kg i.p.); (Psychopharmacology 78, S. 104-111 (1982)) sind die Verbindungen in der Lage, dieser pharmakologisch induzierten cerebralen Insuffizienz entgegenzuwirken bzw. sie aufzuheben.

Bei der Prüfung der Überlebensfähigkeit von Tieren in einer geschlossenen Kammer (Hypoxietoleranztest), welche mit einem Gasgemisch, bestehend aus 96,5 % Stickstoff und 3,5 % Sauerstoff, durchströmt wurde, wiesen die mit den neuen Substanzen vorbehandelten Tiere eine statistisch hochsignifikant größere Überlebensfähigkeit auf als Kontrolltiere bzw. mit Diltiazem, Verapamil oder Nifedipin vorbehandelte Tiere. Die mit dieser Methode geprüfte hirnprotektive Wirkung der Substanzen war bereits bei einer Dosis von 5 mg/kg p.o. ausgeprägt. Damit sind die neuen Verbindungen sowohl hinsichtlich der wirksamen Dosis als auch der tierexperimentell erzielten Leistungsverbesserung den genannten bekannten Substanzen deutlich überlegen.

Aufgrund dieser Befunde sollen die Verbindungen der allgemeinen Formel I bzw. ihre Säureadditionssalze als Wirkstoffe für Arzneimittel gegen das hirnorganische Psychosyndrom sowie posttraumatische und alkoholische Hirnschädigung Verwendung finden.

Die Verbindungen können sowohl enteral als auch parenteral verabreicht werden. Als Dosis für die orale Anwendung werden 2 bis 20 mg/kg, vorzugsweise 5 bis 10 mg/kg Wirkstoff 1 bis 3 mal pro Tag vorgeschlagen.

Geeignete Anwendungsformen sind beispielsweise Tabletten, Kapseln, Zäpfchen, Lösungen, Säfte, Emulsionen, Aerosole oder dispersible Pulver. Entsprechende Zubereitungen können beispielsweise durch Mischen des oder der Wirkstoffe mit bekannten Hilfsstoffen erhalten werden.

A. Ein weiterer Gegenstand der vorliegenden Erfindung besteht aus einem neuen Verfahren, dadurch gekennzeichnet, daß man eine Verbindung der allgemeinen Formel I, worin A die Gruppe (Ic) ist, zur Herstellung einer Verbindung I, worin A die Gruppe (Ia) ist, in Gegenwart von Wasser und einer Protonensäure cyclisiert und gegebenenfalls die erhaltene Verbindung der allgemeinen Formel I in ein Säureadditionssalz überführt oder ein Säureadditionssalz durch Umsalzen in ein anderes Säureadditionssalz überführt.

Besonders vorteilhaft ist das Verfahren für die Herstellung der Verbindungen der Formel I, worin A die Gruppe (Ia) ist und B die Gruppe 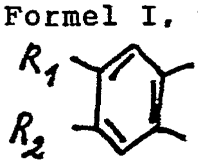 ,

wenn von Verbindung II ausgegangen wird.

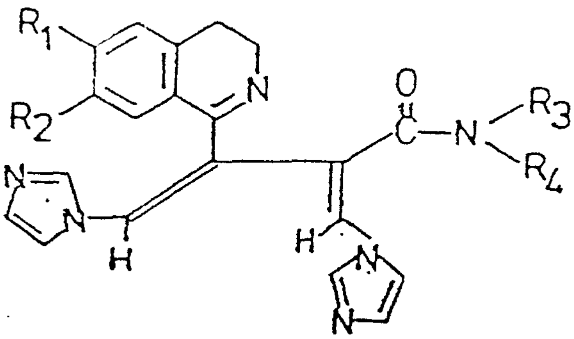

II

Die Reaktionstemperatur ist nicht kritisch. Die Reaktion kann innerhalb eines großen Temperaturbereiches, vorzugsweise unter Erwärmen oder Erhitzen bis zum Siedepunkt des Lösungsmittels, durchgeführt werden.

Die Reaktion kann in jedem Lösungsmittel oder in -gemischen durchgeführt werden, sofern sie eine ausreichende Löslichkeit für die Reaktionskomponenten besitzen und das Reaktionsgeschehen nicht nachteilig beeinflussen, z.B. Ethanol, Methylenchlorid, Chloroform, Tetrahydrofuran und Dioxan.

Als Säuren können alle Protonensäuren, wie z.B. Salzsäure, Schwefelsäure oder Phosphorsäure Anwendung finden.

Normalerweise wird eine verdünnte Säure z.B. 2N Salzsäure verwendet.

B. Ein weiterer Gegenstand der vorliegenden Erfindung besteht aus einem neuen Verfahren zur Herstellung der Verbindung I, worin A die Gruppe der Formel (Ic) ist, wobei man eine Verbindung

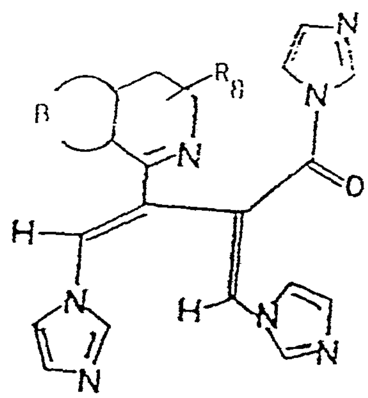

III

in der B und $R_8$ wie ~~in Anspruch 1~~ oben definiert sind, mit einem Amin der Formel IV

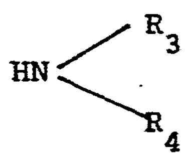

IV

worin $R_3$ und $R_4$ wie ~~in Anspruch 1~~ oben definiert sind, oder einem reaktiven Derivat davon umsetzt.

Die Umsetzung des Imidazolids der Formel III geschieht in an sich bekannter Weise in einem inerten Lösungsmittel bei Raumtemperatur oder erhöhter Temperatur. Die Reaktionstemperatur ist nicht kritisch. Ihre Wahl wird ebenso wie die Reaktionsdauer von der Reaktivität des eingesetzen Amins IV bestimmt. Als Lösungsmittel können jene, die in Bezug auf die Cyclisierungsreaktion erwähnt wurden, z.B. Methylenchlorid, verwendet werden. Falls erwünscht, kann die Verbindung der Formel I, worin A die Gruppe Ic ist, isoliert und anschließend der

Cyclisierungsreaktion unterworfen werden. Bevorzugt wird jedoch das gebildete Produkt direkt d.h. ohne den Zwischenschritt der Isolierung in das Endprodukt der allgemeinen Formel I, worin A die Gruppe (Ia) ist, überführt.

Der Ausgangsstoff der Formel III kann durch Umsetzung einer Furancarbonsäure der Formel V

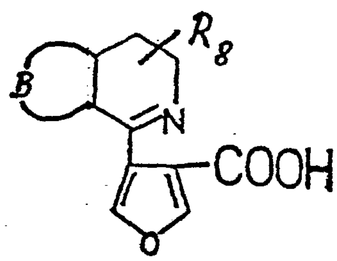

V

in der B und $R_8$ die oben angegebene Bedeutung besitzen, in einem geeigneten Lösungsmittel mit zwei Moläquivalenten von N,N'-Carbonyldiimidazol hergestellt werden. Die Umsetzung kann bei Raumtemperatur oder Temperaturen bis zur Siedetemperatur des eingesetzen Lösungsmittels durchgeführt werden.

Die Reaktionsdauer beträgt im allgemeinen zwischen 1 und 15 Stunden.

Als Lösungsmittel können alle zuvor genannten Solventen oder Gemische davon verwendet werden, solange sie das Reaktionsgeschehen nicht nachteilig beeinflussen, wie z.B. diejenigen, die als geeignet für die Cyclisierungsreaktion beschrieben wurden.

Bevorzugt wird die Reaktion in Methylenchlorid bei Siedetemperatur durchgeführt.

C. Zur Herstellung einer Verbindung I, worin A die Gruppe der Formel (Ib) ist, wird eine Verbindung der Formel I, worin A die Gruppe der Formel (Ia) ist mit einem Amin $NH_2R_7$ (worin $R_7$ wie oben definiert ist) oder einem reaktiven Derivat davon nach Verfahren, die für die Herstellung Schiff'scher Basen bekannt sind, umgesetzt.

## Beispiele

1. 1,4-Di-(1-imidazolyl)-3-[(3,4-dihydro-6,7-dimethoxy)-1-Isochinolinyl-butadien-2-carbonsäure imidazolid

5 g (15 mmol) 4-(3,4-Dihydro-6,7-dimethoxy-1-isochinolinyl)-3-furancarbonsäure-hydrochlorid werden mit 6 g (37 mmol) N,N'-carbonyldiimidazol in 100 ml wasserfreiem Methylenchlorid zwei Stunden im Wasserbad auf Siedetemperatur erwärmt. Nach beendeter Reaktion wird die nunmehr gelborange Lösung bis zur beginnenden Kristallisation vorsichtig eingeengt und das Reaktionsprodukt durch Zugabe von trockenem Ether zur Kristallisation gebracht.

Ausbeute: 6,9 g (83 % d. Th.), feine, gelbe Nadeln, Schmp. 234-236°C.

1,4-Di-(1-imidazolyl)-3-[(3,4-dihydro-6,7-dimethoxy)-1-isochinolinyl]-butadien-2-carboxamid

4,7 g (10 mmol) 1,4-Di-(1-imidazolyl)-3-[(3,4-dihydro-6,7-di-methoxy)-1-isochinolinyl]-butadien-2-carbonsäure imidazolid werden in einer ausreichenden Menge wasserfreien Methylenchlorids gelöst und mit 20 ml einer mit Ammoniak gesättigten Methylenchloridlösung versetzt. Die Mischung wird 4 Stunden bei Raumtemperatur eingeengt, und der Rückstand aus Essigester kristallisiert.

Ausbeute: 3,8 g (93 % d. Th.), gelbe Nadeln,
Schmp. 224-225°C.

In analoger Weise werden erhalten:

1,4-Di-(1-imidazolyl)-3-[(3,4-dihydro-6,7-dimethoxy)-1-isochinolinyl]-N-(2-furylmethyl)-butadien-2-carboxamid.

Ausbeute: 67 % d. Th., gelbe Nadeln, Schmp. 212-217°C.

1,4-Di-(1-imidazolyl)-3-[(3,4-dihydro-6,7-dimethoxy)-1-isochinolinyl]-N,N-di-(n-propyl)-2-carboxamid,

Ausbeute: 88 % d. Th., gelbe Nadeln, Schmp. 125-127°C

1,4-Di-(1-imidazolyl)-3-[3,4-dihydro-6,7-dimethoxy)-1-isochinolinyl]-N-(1,5-dimethylhexyl)-2-carboxamid.

Ausbeute: 84 % d. Th., gelbe Nadeln, Schmp. 119-120°C.

Beispiel 2A

2-(Furan-2-yl-methylamino)-4-[(3,4-dihydro-6,7-dimethoxy)-1-isochinolinyl]-furan-3-carbaldehyd

Eine Lösung von 1,4-Di-(1-imidazolyl)-3-[(3,4-dihydro-6,7-dimethoxy)-1-isochinolinyl]-N-(2-furylmethyl)-butadien-2-carboxamid (5 g; 10 mmol) in 100 ml Ethanol wird mit 2N Salzsäure (10 ml) versetzt und ca. 1 Stunde auf 50°C erwärmt. Danach wird mit Sodalösung neutralisiert, das Reaktionsprodukt mit Methylenchlorid extrahiert, über eine $Al_2O_3$-Säule (neutral, Aktivitätsstufe III, Eluent: $CH_2Cl_2$) gereinigt und aus Essigester/Petroläther kristallisiert.

Ausbeute: 3,1 g (82 % d. Th.) gelbgrüne Nadeln. Schmp. 163-164°C.

Beispiel 2B

2-(Furan-2-yl-methylamino)-4-[(3,4-dihydro-6,7-dimethoxy)-1-isochinolinyl]-furan-3-carbaldehyd

a) Bildung des Ausgangsstoffes

4-[3,4-Dihydro-6,7-dimethoxy)-1-isochinolinyl]-furan-3-carbonsäure-hydrochlorid (5 g; 15 mmol) wird mit N,N'-Carbonyldiimidazol (4,9 g; 30 mmol) in wasserfreiem $CH_2Cl_2$ (100 ml) 1-2 Stunden auf Siedetemperatur erwärmt.

b) Herstellung des Endprodukts

Nach Bildung des Imidazolids wird 2-(Aminomethyl)-furan (1,5 g 15 mmol) zugegeben, wird 6 Stunden bei Raumtemperatur gerührt, danach mit Ethanol (100 ml) verdünnt, mit 2N Salzsäure (10 ml) versetzt und ca. 1 Stunde auf 50°C erwärmt. Nach der Beispiel 1 analogen Aufarbeitung des Reaktionsansatzes wird aus Essigester/Petroläther kristallisiert.

Ausbeute: 4,6 g (76 % d. Th.), gelbe Nadeln,
Schmp. 163-164°C.

Beispiele 3-34

Auf analoge Weise, wie in Beispiel 2A oder in Beispiel 2B beschrieben, werden die nachstehenden Verbindungen erhalten.

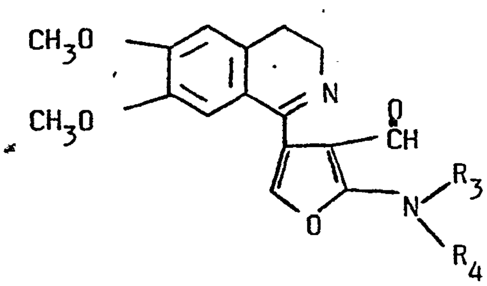

| Beispiel | $R_3$ | $R_4$ | Fp°C | Ausb. % |
|---|---|---|---|---|
| 3 | H | H | 220-222 | 93 |
| 4 | H | $CH_3-$ | 213-214 | 82 |
| 5 | H | $C_2H_5$ | 189-195 | 76 |
| 6 | H | $CH_3-(CH_2)_2-$ | 190-192 | 65 |
| 7 | H | $CH_3-(CH_2)_4-$ | 139-141 | 69 |
| 8 | H | $(CH_3)_2-CH-$ | 184-185 | 71 |
| 9 | H | $(CH_3)_2-CH-CH_2-$ | 145-148 | 82 |
| 10 | H | $(CH_3)_2-CH-(CH_2)_2-$ | 138-139 | 69 |
| 11 | H | $C_2H_5-CH(CH_3)-CH_2-$ | 119-120 | 73 |
| 12 | H | $CH_3-(CH_2)_2-CH(CH_3)-$ | 145-147 | 79 |
| 13 | H | $(CH_3)_3\ C-$ | 186-188 | 67 |
| 14 | H | $CH_3-CH(CH_3)-CH(CH_3)$ | 118-125 | 65 |
| 15 | H | $CH_2=CH-CH_2-$ | 151-153 | 71 |
| 16 | H | $CH\equiv C-CH_2-$ | 211-213 | 75 |
| 17 | H | 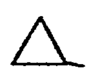 | 192-196 | 64 |
| 18 | H | 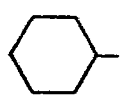 | 172-173 | 62 |
| 19 | H | $HO-(CH_2)_2-$ | 187-194 | 70 |
| 20 | H | $CH_3O-(CH_2)_2-$ | 143-145 | 69 |
| 21 | H | $CH_3O-(CH_2)_3-$ | 139-140 | 67 |
| 22 | H | $(CH_3)_2N-(CH_2)_2-$ | 162-164 | 59 |
| 23 | H | $(CH_3)_2N-(CH_2)_3-$ | 142-143 | 61 |

| | | | | |
|---|---|---|---|---|
| 24 | H | 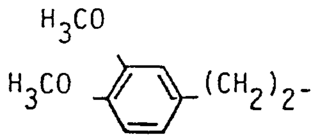 | 164-167 | 78 |
| 25 | H | 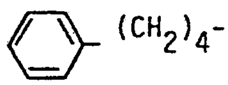 | 126-127 | 67 |
| 26 | H | 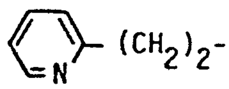 | 162-165 | 68 |
| 27 | H | 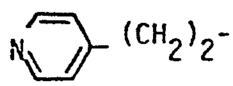 | 173-176 | 72 |
| 28 | H | 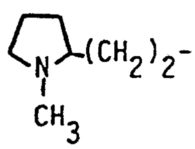 | 119-122 | 69 |
| 29 | $CH_3$ | $CH_3$ | 217-219 | 67 |
| 30 | $C_2H_5$ | $C_2H_5$ | 125-130 | 70 |
| 31 | $CH_3-(CH_2)_2-$ | $CH_3-(CH_2)_2-$ | 146-147 | 73 |
| 32 | $CH_3-(CH_2)_3-$ | $CH_3-(CH_2)_3-$ | 85- 89 | 75 |
| 33 | $C_2H_5$ | $CH_3-(CH_2)_3-$ | 148 | 67 |
| 34 | $-CH_2-CH_2-CH_2-CH_2-$ | | 216-217 | 58 |

Auf analoge Weise, wie in Beispiel 1 beschrieben, werden Verbindungen der Formel

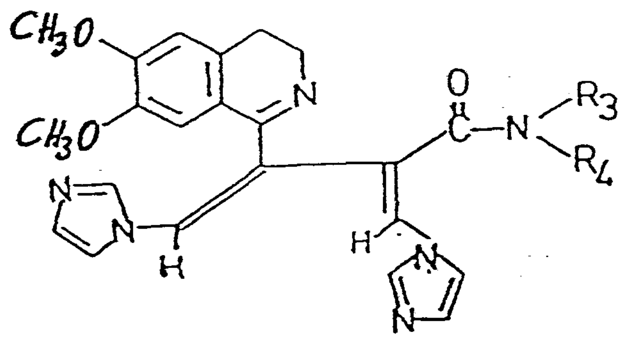

erhalten, in denen $R_3$ und $R_4$ wie in der obigen Tabelle definiert sind.

Durch Umsetzung der Verbindungen der Beispiele 1 bis 34, nach für die Herstellung Schiff'scher Basen bekannten Methoden, werden die entsprechenden Verbindungen der Formel

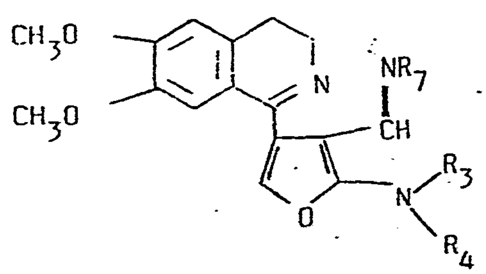

erhalten, wie zum Beispiel

| $R_4$ | $R_3 = R_7$ | Fp. |
|---|---|---|
| H | $-CH_2-C_6H_5$ | 116-118°C |
| H | 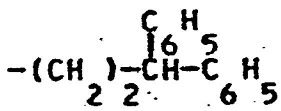 | 156-158°C |
| H | 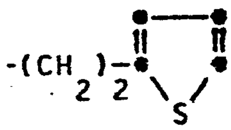 | 142-144°C |
| H | $-C_6H_5$ | 199-220°C |

0234329

Patentansprüche

1) Verbindungen der allgemeinen Formel I

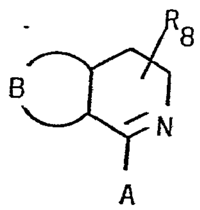

oder ihr Säureadditionssalz
worin,

A die Gruppe Ia, Ib oder Ic ist,

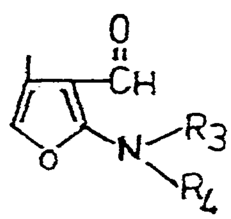

Ia

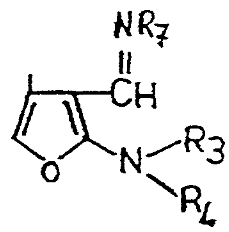

Ib

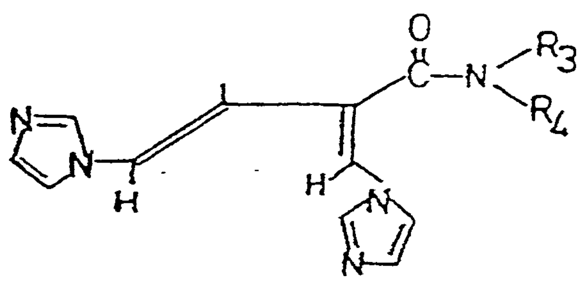

Ic

B zusammen mit den beiden vicinalen C-Atomen ein aromatisches System

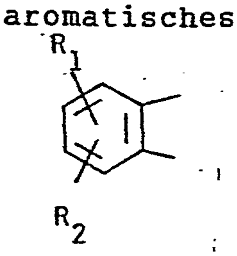

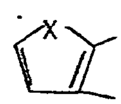

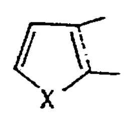

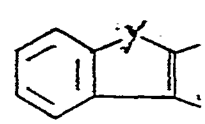

oder

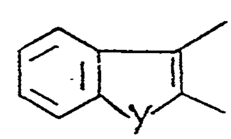

bedeutet,
worin
X S oder $NR_5$ (worin $R_5$ H, Methyl oder Benzyl ist) bedeutet;
Y S, O oder $NR_6$ (worin $R_6$ wie $R_5$ definiert ist) bedeutet;

0234329

$R_1$ und $R_2$ unabhängig voneinander Wasserstoff ($C_1$-$C_4$)-Alkoxy, Mercapto, Methylmercapto oder Amino oder zusammen die Gruppe 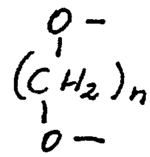

worin n 1 oder 2 ist, bedeuten;

$R_3$ und $R_4$ unabhängig voneinander (i) Wasserstoff, oder (ii) einen gesättigten ($C_1$-$C_{12}$) oder ungesättigten ($C_2$-$C_{12}$), geradkettigen oder verzweigten oder cyclischen ($C_3$-$C_6$) Kohlenwasserstoffrest, der gegebenenfalls ein- oder mehrfach durch Hydroxy, Methoxy, Amino, ($C_1$-$C_5$)-Alkylamino, Di- ($C_1$-$C_5$)-alkylamino, Phenyl (gegebenenfalls ein- oder mehrfach durch Hydroxy, Methoxy oder Halogen substituiert), oder durch einen 5- oder 6-gliedrigen gesättigten oder ungesättigten Heterocyclus (der ein oder zwei N, S oder O Heteroatome enthält, und der durch ($C_1$-$C_3$) Alkyl substituiert sein kann) substituiert ist,

oder $R_3$ und $R_4$ zusammen mit dem Stickstoffatom einen 5- oder 6-gliedrigen Ring bilden, der gegebenenfalls ein weiteres N, O oder S Heteroatom enthält, bedeuten;

$R_7$ wie $R_3$ definiert ist;

und $R_8$ H, ($C_1$-$C_3$)-Alkyl oder ($C_3$-$C_6$)-Cycloalkyl oder $-SCH_3$ bedeutet.

2) Verbindungen nach Anspruch 1, worin B zusammen mit den vicinalen C-Atomen die Gruppe 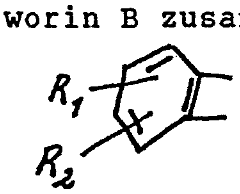

bedeutet.

3) Verbindungen nach Anspruch 2, worin $R_1$ und $R_2$ in Position 6 beziehungsweise 7 sind.

4) Verbindung nach Anspruch 2 oder 3, worin $R_1$ und $R_2$ Methoxy sind.

5) Verbindung nach einem der Ansprüche 1 bis 4, worin $R_3$ für Wasserstoff und $R_4$ für Methyl-, Ethyl-, Allyl-, Propargyl-, geradkettige oder verzweigte $(C_3-C_5)$alkyl-, Methoxy $(C_1-C_4)$alkyl- oder Hydroxy $(C_1-C_4)$alkyl steht.

6) Verbindung nach einem der Ansprüche 1 bis 4, worin $R_3$ für Wasserstoff und $R_4$ für ($C_3$ bis $C_6$)Cycloalkyl oder Phenyl steht.

7) Verbindung nach einem der Ansprüche 1 bis 4, worin $R_3$ und $R_4$ unabhängig voneinander für eine $(C_1-C_5)$-Alkylgruppe stehen.

8) Verbindung nach einem der Ansprüche 1 bis 4, worin $R_3$ für H und $R_4$ für $CH_3$ oder $(CH_3)_2-CH-(CH_2)_2-$ oder $C_2H_5-CH(CH_3)-CH_2-$ oder $(CH_3)_3-C-$ steht.

9) Verbindung nach einem der Ansprüche 1 bis 4, worin $R_3$ und $R_4$ für $C_2H_5$ stehen oder zusammen die Gruppe $-(CH_2)_4-$ bilden.

10) Verbindung nach einem der Ansprüche 1 bis 9, worin A die Gruppe der Formel (Ia) ist.

11) Pharmazeutische Zubereitungen, enthaltend Substanzen nach einem der Ansprüche 1 bis 10 in Kombination mit üblichen Hilfs- und/oder Trägerstoffen.

12) Verbindungen nach einem der Ansprüche 1 bis 10 zur Verwendung bei der Behandlung der koronaren Herzkrankheit oder cerebraler Stoffwechselstörungen.

13) Verfahren zur Herstellung einer Verbindung gemäß einem der Ansprüche 1 bis 10, dadurch gekennzeichnet, daß man

A. (zur Herstellung einer Verbindung I, worin A die Gruppe der Formel (Ia) ist) eine Verbindung der Formel I, worin A die Gruppe der Formel (Ic) ist, in Gegenwart von Wasser und einer Protonensäure cyclisiert;

B. (zur Herstellung einer Verbindung I, worin A die Gruppe der Formel (Ic) ist), eine Verbindung

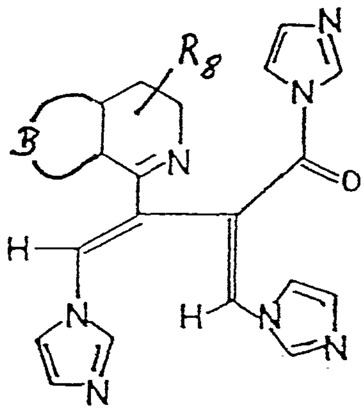

III

in der B und $R_8$ wie in Anspruch 1 definiert sind, mit einem Amin der Formel IV

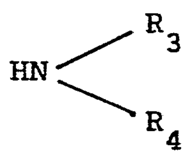

IV

worin $R_3$ und $R_4$ wie in Anspruch 1 definiert sind, oder einem reaktiven Derivat davon umsetzt;

C. (zur Herstellung einer Verbindung I, worin A die Gruppe der Formel (Ib) ist), eine Verbindung der Formel I, worin A die Gruppe der Formel (Ia) ist, mit einem Amin $NH_2R_7$ (worin $R_7$ wie in Anspruch 1 definiert ist) oder einen reaktiven Derivat davon umsetzt;

und gegebenenfalls die erhaltene Verbindung der allgemeinen Formel I in ein Säureadditionssalz überführt oder ein Säureadditionssalz durch Umsalzen in ein anderes Säureadditionssalz überführt.

Europäisches Patentamt

# EUROPÄISCHER RECHERCHENBERICHT

Nummer der Anmeldung 0234329

EP 87 10 1292

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| A | EP-A-0 078 949 (BOEHRINGER INGELHEIM KG)<br>* Ansprüche 1, 8-10 *<br>--- | 1,11,<br>12 | C 07 D 405/04<br>C 07 D 405/14<br>C 07 D 401/14<br>C 07 D 409/14<br>C 07 D 471/04 |
| A | EP-A-0 150 474 (BOEHRINGER INGELHEIM KG)<br>* Seite 4, Formel II *<br>----- | 1 | C 07 D 491/04<br>C 07 D 495/04<br>A 61 K 31/47 //<br>(C 07 D 471/04<br>C 07 D 221:00<br>C 07 D 209:00 )<br>(C 07 D 491/04<br>C 07 D 307:00<br>C 07 D 221:00 )<br>(C 07 D 491/04<br>C 07 D 317:00<br>C 07 D 221:00 )<br>(C 07 D 491/04<br>-/- |

RECHERCHIERTE SACHGEBIETE (Int. Cl.4)

C 07 D 405/00
C 07 D 401/00
C 07 D 409/00
C 07 D 521/00
C 07 D 471/00
C 07 D 491/00
C 07 D 495/00
A 61 K 31/00

Der vorliegende Recherchenbericht wurde für alle Patentansprüche erstellt.

| Recherchenort | Abschlußdatum der Recherche | Prüfer |
|---|---|---|
| BERLIN | 05-05-1987 | VAN AMSTERDAM L.J.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82

Europäisches Patentamt

# EUROPÄISCHER RECHERCHENBERICHT

Nummer der Anmeldung 0234329

EP 87 10 1292

Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| | | | C 07 D 319:00<br>C 07 D 221:00 )<br>(C 07 D 495/04<br>C 07 D 333:00<br>C 07 D 221:00 ) |
| | | | RECHERCHIERTE SACHGEBIETE (Int. Cl.4) |

Der vorliegende Recherchenbericht wurde für alle Patentansprüche erstellt

| Recherchenort | Abschlußdatum der Recherche | Prüfer |
|---|---|---|
| BERLIN | 05-05-1987 | VAN AMSTERDAM L.J.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82